# EUROPÄISCHE PATENTSCHRIFT

(11) **EP 0 291 630 B1**
(45) Veröffentlichungstag und Bekanntmachung des Hinweises auf die Patenterteilung: **28.04.1993**
(21) Anmeldenummer: 88101569.7
(22) Anmeldetag: 04.02.1988
(51) Int. Cl.: B01D 53/34, G01N 1/34

(54) **Verfahren und Einrichtung zur Aufbereitung eines zu analysierenden Gases**
Process and apparatus for pretreatment of a gas to be analysed
Procédé et appareil pour prétraiter un gaz à analyser

(30) Priorität: 15.05.1987 DE 3716350
(43) Veröffentlichungstag der Anmeldung: 23.11.1988
(73) Patentinhaber: Gröger & Obst Mess- und Regeltechnik GmbH, D-82335 Berg (DE)
(72) Erfinder: Gröger, Helmut, D-8035 Stockdorf (DE)
(74) Vertreter: LOUIS, PÖHLAU, LOHRENTZ & SEGETH

(56) Entgegenhaltungen:
- DE-A- 2 031 298
- DE-A- 2 450 811
- FR-A- 2 212 925
- US-A- 3 304 783
- US-A- 4 612 019
- CHEMIE, INGENIEUR, TECHNIK, Band 53, Nr. 9, September 1981, Seiten 731-733, Nürnberg, DE; F. WODTKE:"Betriebserfahrungen mit Perma-Pure-Trockenrohren zum Entfernen von Wasser aus Gasen oder Flüssigkeiten"

## Beschreibung

Die Erfindung betrifft ein kontinuierliches Verfahren zur Aufbereitung eines zu analysierenden Gases. Weiterhin betrifft die Erfindung eine Einrichtung zur Durchführung dieses Verfahren.

Die Methoden zur Feststellung, ob und in welcher Menge gewisse Stoffe in einem Gas, z.B. Abgas, enthalten sind, erfordern es, aus dem zu analysierenden Gas zunächst solche Bestandteile abzuscheiden, die diese Feststellung beeinträchtigen und das Analyseergebnis verfälschen. So ist es z.B. notwendig, bei der Analyse von Verbrennungsabgasen aus Kraftwerken, Müllverbrennungsanlagen usw., durch welche z.B. der Gehalt an SO₂, CO usw. festgestellt werden soll, zunächst andere Stoffe, z.B. SO₃, aus dem zu analysierenden Gas zu entfernen. Denn solche Stoffe ergeben entweder bei der Messung der zu analysierenden Zielsubstanz eine Querempfindlichkeit oder sie lösen die Zielsubstanz teilweise und entziehen sie damit der Analyse, wie das für Wasser der Fall ist. Insbesondere Wasser, das in heissen Abgasen in Form von Dampf vorhanden ist, muß vollständig und auf eine Weise aus dem Gas abgeschieden werden, daß es nicht in der Lage, die ggf. wasserlösliche Analyse-Zielsubstanz in merklichen Mengen zu lösen, wenn das Analyseergebnis richtig sein soll. Bei den bekannten Verfahren zur Aufbereitung von zu analysierenden Gasen wird zum zweck der Beseitigung des Wasserdampfes das Gas zunächst abgekühlt, um den Wasserdampf zum Kondensieren zu bringen und das Kondensat auszuscheiden. Um auf diese Weise das Wasser möglichst weitgehend zu entfernen, ist es bekannt, die Abkühlung mehrstufig durchzuführen, um jeweils nach der Teilentfernung von Kondensat den Taupunkt zu unterschreiten. Es ist auf diese Weise jedoch bisher nicht gelungen, eine Restmenge von etwa 5 g/m³ Wasserdampfgehalt zu unterschreiten. Überdies weist der hierzu erforderliche Mehrstufenkühler ein verhältnismässig grosses Volumen auf (das bis zu 500 l betragen kann), welches das zur Verfügung stehende Volumen an Analysegas z.T. erheblich überschreitet, so daß die Messung entweder nicht ausgeführt werden kann oder eine sehr lange Meßdauer (90%-Zeit) erfordert.

Bei der Messung mit Infrarot-Meßgeräten kommen Meßfehler hinzu, die infolgendes bei der Mehrstufen-Abkühlung entstehenden Gaszustandes auftreten. Da bisher in der Praxis eine weitergehende Entfernung des Wassers aus dem zu analysierenden Gas nicht als möglich angesehen worden ist, hat man die aus dem verbleibenden Wasserdampfgehalt von etwa 5 g/m³ resultierenden Meßfehler in Kauf genommen.

Bei einem bekannten Verfahren zum Entfernen von Wasser aus Gasen kommt ein Permeationstrockner zum Einsatz, wobei vor der Hindurchleitung des Gases durch den Permeationstrockner zunächst kondensierbare Stoffe wie Öl, Säuredämpfe und dgl. entfernt werden müssen (Chemie Ingenieur Technik Bd. 53(1981) Nr. 9). Die Verwendung des Permeationstrockners setzt voraus, daß auch Wassertröpfchen und ggf. vorhandene Laugesprüh aus Abgaswäschen vor dem Eintritt des Gases in den Permeationstrockner zu entfernen sind. Jedoch lässt sich der Permationstrockner, insbesondere bei höheren Wasserdampfgehalten in dem zu analysierenden Gas, ohne vorherige Behandlung des Analysegases nicht einsetzen, ohne daß die Funktion des Permeationstrockners gefährdet wird. Denn die im Gas enthaltenen weiteren Bestandteile, die auch als Aerosol vorliegen können, beeinträchtigen den Permeationstrockner in seiner Funktion und machen diesen u.U. bereits nach kurzer Betriebsdauer unbrauchbar.

Der Erfindung liegt daher die Aufgabe zugrunde, ein kontinuierliches Aufbereitungsverfahren und eine Einrichtung hierfür zu schaffen, die es trotz geringerem apparativen Aufwand erlauben, ein zu analysierendes Gas von Wasser möglichst vollständig zu befreien. Unter kontinuierlich wird in diesem Zusammenhang eine Vorgangsweise verstanden, bei der während des eigentlichen Aufbereitungsvorganges das Gas ohne Zwischenspeicherung die Einrichtung durchströmt, um für das eigentliche Analyseverfahren bereitgehalten zu werden.

Diese Aufgabe wird gelöst durch das Verfahren gemäß Patentanspruch 1. Die hierzu erforderliche Einrichtung ist im Patentanspruch 4 angegeben.

Die Erfindung geht von der Erkenntnis aus, daß es zur vollständigen Abscheidung des Wassers einer bestimmten Kombination und der Einhaltung einer bestimmten Reihenfolge der hierzu durchgeführten Verfahrensschritte bedarf, um das Wasser tatsächlich zu entfernen. Zunächst erfolgt deshalb eine Abkühlung des Gases, vorzugsweise bis auf etwa 4°C, durch die in der Regel der Taupunkt der im Gas enthaltenen Wasserdampfmenge soweit unterschritten wird, daß ein beträchtlicher Anteil des Wassers als Kondensat abgeführt werden kann. Da im Zuge der Abkühlung aber ein Teil des Wasserdampfes nicht in Form von Kondensat, sondern als Nebel, d.h. als Aerosol, ausfallen kann und daher nicht mit dem Kondensat abgeführt wird, ist als nächster Schritt die Beseitigung dieses Aerosols erforderlich. Danach ist, wie eingangs erläutert, in dem Gas eine Restmenge von etwa 5 bis 8 g/m³ als Wasserdampf enthalten, die nunmehr - nachdem weitere störende Bestandteile, darunter auch Festkörper, entfernt sind - durch eine Permeationsdestillation entfernt werden. Wesentlich für den von dem erfindungsgemässen Verfahren erzielten Erfolg ist somit die Kombination einer Kühlung, einer Aerosol-Filtrierung und einer Trocknung des Gases und deren Reihenfolge.

Um zu vermeiden, daß das beim Abkühlvorgang als Kondensat austretende Wasser die Analyse-Zielsubstanz oder -substanzen in merklicher Menge löst und damit der Messung entzieht, ist im Rahmen des Verfahrens vorgesehen, daß das Kondensat kontinuierlich abgesaugt wird. Hierdurch wird vermieden, daß sich zwischen Kondensat und strömendem Gas eine grössere Grenzfläche, z.B. im Bereich von Kondensatzansammlungen, ausbildet, über welche Gasbestandteile in das Kondensat eintreten können. Einrichtungsmässig ist es im Rahmen der Erfindung hierzu möglich, die Abkühlung mit einer Kühlschlange durchzuführen, wenn durch die erwähnte Absaugung des Kondensats dafür gesorgt werden kann, daß Kondensat nicht in merklichen Mengen an der Innenwandung der Kühlschlange herablaufen kann. Bevorzugt findet hierzu nach der Erfindung ein Kühler mit mindestens zwei konzentrischen Rohren Anwendung, die ineinander münden und das Kondensat getrennt freigeben.

Das hat den Vorteil, daß das Kondensat auf sehr kurzem Wege aus dem Gas austritt und zum unteren Ende des Entspannungskühlers hin abtropft, wo es durch Absaugung sofort beseitigt werden kann. Das Kondensat, das auch weitere und teils aggressive Bestandteile enthält, die darin gelöst sind, wird zu einem Kondensatbehälter gefördert, in welchem es einer späteren Entsorgung zugeführt werden kann.

Das Aerosolfilter ist von einer Art, daß es bei Kontakt mit den im Gasstrom mitgeführten Flüssigkeitströpfchen diese dem Gasstrom entzieht und an ihn keine Feuchtigkeit mehr abgibt. Hierzu muß es aus einem netzfähigen und zweckmässigerweise feuchtigkeitsaufnehmenden Material bestehen, das die Feuchtigkeit festhält. Geeignet hierfür ist ein Fasermaterial oder Vlies aus Baumwollfasern, Acrylfasern.

Die Trocknung des Gases durch Permeationsdestillation erfolgt mittels eines an sich bekannten Permeationstrockners, in dem das Gas durch einen oder mehrere Schläuche geleitet wird, der außen von einem Gegenstrom aus trockenem Gas, z.B. Luft, bestrichen ist. Zur Führung des Gegenstromes ist der erstgenannte gasführende Schlauch von einem weiteren Schlauch umschlossen. Das Material des gasführenden inneren Schlauches ist derart gewählt, daß der Wasserdampf durch dessen Wandung unmittelbar in Dampfform hindurchtreten und durch das außen strömende trockene Gas abgeführt werden kann.

Entweder vor oder hinter dem Aerosolfilter können ein oder mehrere chemische Filter angeordnet sein, die unerwünschte chemische Bestandteile aus dem Gasstrom entfernen. Insbesondere bei der katalytischen Beseitigung von SO₂ aus Verbrennungsabgasen hat sich gezeigt, daß in zunehmendem Maße solche Abgase auch SO₃ enthalten, das die SO₂-Analyse beeinträchtigt. Ein wesentlicher Gedanke des erfindungsgemässen Verfahrens, für den gesondert Schutz beansprucht wird, besteht in der Erkenntnis, daß der SO₃-Gehalt des Gases durch ein Opfermetallfilter in Form von Stahlwolle beseitigt werden kann. Andere Bestandteile des Gases, z.B. Halogenide, können durch Opfermetallfilter aus Zink oder Bronze, in Pulver- oder Gitterform, entfernt werden.

Es versteht sich, daß im Rahmen des erfindungsgemässen Verfahrens auch Maßnahmen vorgesehen sind oder sein können, die zur Beseitigung ggf. in dem Gas zu erwartender Festkörperpartikel, wie Rußteilchen, dienen. Diese sind bezüglich ihrer Anordnung in der Reihenfolge der vorstehend beschriebenen Maßnahmen zur Beseitigung des Wasserdampfes weniger kritisch und können beispielsweise bereits zu Beginn des Aufbereitungsprozesses durchgeführt werden. Auch versteht sich, daß zusätzliche chemische Vorgänge eingeschaltet sein können, die zur Beseitigung unerwünschter anderer Bestandteile dienen, z.B. eine Ammoniakauswaschung.

Aufgrund der geschilderten Abfolge der Verfahrensschritte bei der Beseitigung des Wasserdampfes aus dem aufzubereitenden Gas ist es möglich, die für den Analysevorgang notwendigen Gasvolumina erheblich kleiner als bisher zu halten, so daß dementsprechend auch die für die Durchführung des erfindungsgemäßen Verfahrens notwendige Einrichtung klein gehalten werden kann. So lässt sich diese Einrichtung ohne Schwierigkeit in einem tragbaren Koffer unterbringen, so daß jetzt der vor jeder Analyse notwendige Aufbereitungsvorgang unmittelbar an Ort und Stelle durchgeführt werden kann und aufgrund der niedrigen Totvolumina (niedrige 90%-Zeit) in sehr kurzen Zeitabständen aufeinanderfolgende Messungen durchgeführt werden können.
Weitere Vorteile und Merkmale der Erfindung ergeben sich aus der nachfolgenden Beschreibung bevorzugter Ausführungsbeispiele anhand der beiliegenden Zeichnungen sowie aus weiteren Unteransprüchen. In den Zeichnungen zeigen:
- Fig. 1: schematisch in Form eines Gaslaufplanes die für die Durchführung des Verfahrens erforderliche Einrichtung, und
- Fig. 2 bis 5: schematische Darstellungen bevorzugter Ausführungsformen von einigen der in der Einrichtung gemäß Fig. 1 verwendeten Komponenten, nämlich eines Kühlers, einer Kondensatfalle, eines kombinierten Aerosol- und Opfermetallfilters und des Permeationstrockners.

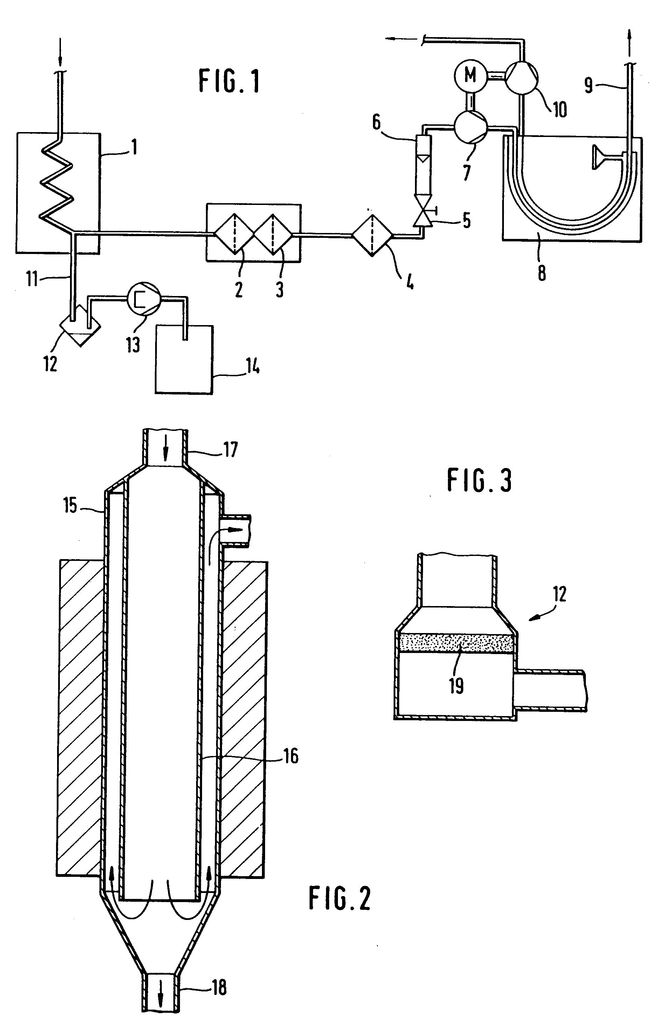

Wie aus dem Gaslaufplan gemäß Fig. 1 hervorgeht, umfasst die erfindungsgemässe Einrichtung einen Gaskühler 1, ein im Gasstrom liegendes Aerosolfilter 2, ein daran anschließend angeordnetes Opfermetallfilter 3 und ein Membran-Feinfilter 4 zur Beseitigung feiner und feinster Festkörperteilchen. Der aus dem Feinfilter 4 austretende Gasstrom wird mittels eines Mengenregelventils 5 auf einen vorbestimmten Durchsatz dosiert, der an einem Durchfluß-Meßgerät 6 unmittelbar abgelesen werden kann. Gefördert wird der Gasstrom durch eine von einem Motor M betriebenen Saugpumpe 7, die ihn einem Permeationstrockner 8 zuführt. Das am Ausgang 9 des Permeationstrockners 8 austretende völlig trockene Gas wird einer nicht dargestellten und hier nicht bedeutsamen Meß- und Analyseapparatur zugeführt. Durch eine weitere Saugpumpe 10 wird trockene Umgebungsluft in den Permeationstrockner 8 eingesaugt und nach Beladung mit dem Wasserdampfgehalt des zu analysierenden Gases auf der Druckseite der Saugpumpe 10 ins Freie gefördert.

Der Gaskühler 1 steht über eine Kondensatablaufleitung 11 und eine Kondensatfalle 12 mit einer Saugpumpe 13, z.B. einer Schlauchpumpe, in Verbindung, durch die in dem Gaskühler 1 anfallendes und durch die Gasablaufleitung der Kondensatfalle zugeführtes Kondensat augenblicklich weggefördert wird. Das Kondensat gelangt in einen Kondensatbehälter 14, der lösbar mit der Pumpe 13 verbunden ist.

Gemäß Fig. 2 weist der Gaskühler 1 einen von einem Peltier-Kühlelement direkt von außen gekühlten Mantel 15 und ein darin konzentrisch angeordnetes Innenrohr 16 auf, das einen oberen Gasanschluß 17 bildet und kurz vor dem kegelig zulaufenden Unterteil des Mantels 15 frei mündet. Ausfallendes Kondensat bildet an der Innenwand des Mantels 15 und am unteren Rand des Innenrohres 16 Tropfen, die nach unten in den kegelig zulaufenden Unterteil des Mantels 15 fallen und dort über einen Auslaß 18 den Gaskühler 1 augenblicklich verlassen.

Das Kondensat gelangt über die Kondensatablaufleitung 11 in die Kondensatfalle 12, für die eine besonders vorteilhafte Ausführungsform in Fig. 3 gezeigt ist. Diese enthält ein Abschirmelement in Form einer dicht eingepassten Glasfritte 19, durch die aufgrund des darunter herrschenden Ansaugdruckes das zugeführte Kondensat hindurchgesaugt wird. Die Glasfritte 19 verhindert die Ausbildung einer freien Grenzfläche zwischen der Kondensatansammlung in der Kondensatfalle 12 und dem das Innenrohr 16 nach oben verlassenden Gas.

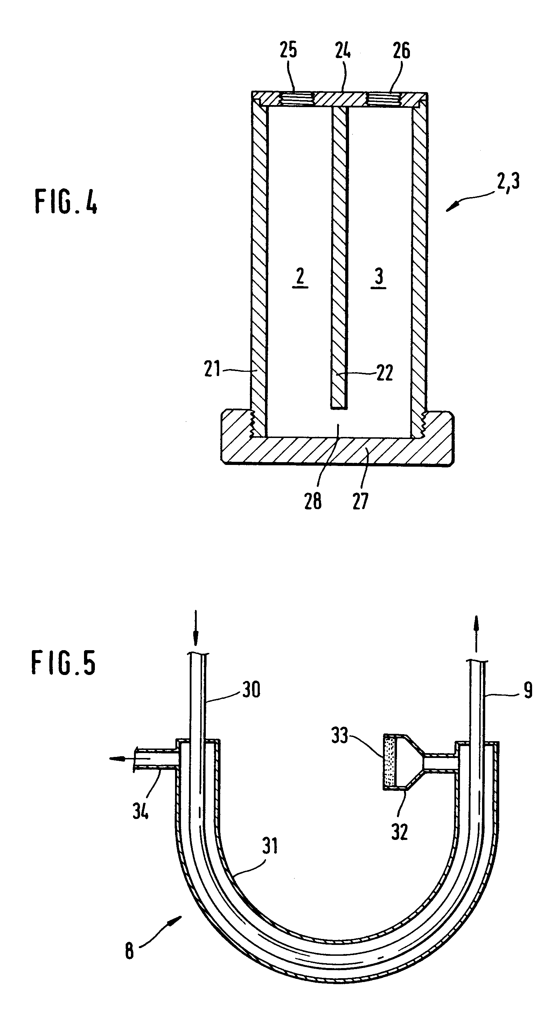

Das Aerosolfilter 2 und das Opfermetallfilter 3 sind zu einem gemeinsamen Filter gemäß Fig. 4 zusammengefasst. Dieses weist einen beispielsweise zylindrischen Behälter 21 auf, der durch eine Trennwand 22 in einen (linken) Filterraum 2 und einen (rechten) Filterraum 3 unterteilt ist. An seinem oberen Ende ist der Behälter 21 durch einen Deckel 24 dicht verschlossen, der eine Gaseintrittsöffnung 25 und eine Gasaustrittsöffnung 26 aufweist. Das untere Ende des Behälters 21 ist durch einen Schraubdeckel 27 verschlossen, der ein Auswechseln der in den Filterräumen 2 und 3 enthaltenen Filterstoffe ermöglicht. Die Trennwand 22 lässt an ihrem unteren Ende zwischen den beiden Filterräumen 2 und 3 einen Gasdurchtritt 28 frei. Der das Aerosolfilter bildende Filterraum 2 enthält eine Faserpackung aus Baumwolle, während in dem Filterraum 3 für den Fall, daß beispielsweise SO₃ abgeschieden werden soll, eine Packung aus Stahlwolle vorgesehen ist.

Die Fig. 5 zeigt schematisch das Bauprinzip des Permeationstrockners 8. Dieses sieht einen für Wasserdampf permeablen Schlauch 30 vor, der von einem äußeren Schlauch 31 umgeben ist. Der äußere Schlauch 31 weist einen Lufteinlaß 32 auf, der als Filterelement eine Glasfritte 33 enthält, sowie einen Sauganschluß 34, an den die Saugpumpe 10 angeschlossen ist. Das Bau- und Wirkprinzip eines derartigen Permeationstrockners ist bekannt und bedarf deshalb an dieser Stelle keiner ins Einzelne gehenden Erläuterung.

Das erfindungsgemässe Verfahren läuft unter Verwendung der in den Zeichnungen gezeigten Einrichtung folgendermassen ab:
Aus einem nicht näher gezeigten Abgasstrom, beispielsweise aus einer Müllverbrennungsanlage, wird ein Teilstrom zum Zweck der Analyse abgezweigt. Dieser Teilstrom wird dem Gaskühler 1 über den Gasanschluß 17 zugeführt und kühlt sich durch Kontakt zunächst mit dem Innenrohr 16 und dann mit dem Mantel 15 von einer Temperatur von beispielsweise 120°C auf beispielsweise 4°C ab. In dem Innenrohr 16 ausfallendes Kondensat sammelt sich in Form von Tropfen am unteren Rand des Innenrohres 16, fällt in den trichterförmigen Unterteil des Mantels 15 und verlässt somit den Strömungsbereich des Gases.An der Innenwand des Mantels 15 ausfallendes Kondensat läuft daran herunter und gelangt ebenfalls in den trichterförmigen Unterteilt. Da unter der Glasfritte 19 der Kondensatfalle 12 aufgrund der durch die Glasfritte bewirkten Drosselung ein gewisser Unterdruck herrscht, werden die Kondensattropfen augenblicklich durch die Glasfritte 19 hindurchgesaugt, so daß eine freie Grenzfläche zwischen dem im Gaskühler befindlichen Gas und einer Kondensatansammlung nicht entsteht.

Durch die Abkühlung wird das Gas von etwa 90% des enthaltenen Wasserdampfes befreit. Es enthält jedoch weiterhin einen Restgehalt von Wasserdampf von etwa 5-8 g/m³ sowie etwa weitere 5 g/m³ Wasser in Form eines Aerosols, das sich im Gaskühler 1 gebildet hat. Das Gas wird am unteren Ende des Innenrohres 16 umgelenkt und verlässt den Gaskühler 1 über den oberen Gasauslaß aufgrund der Saugwirkung der Pumpe 7. Es gelangt zu dem Aerosolfilter 2, das das gebildete Aerosol vollständig aus dem Gas ausscheidet. Die Aerosoltröpfchen werden von den Baumwollfasern des Aerosolfilters aufgenommen und beladen dieses im Laufe der Zeit mit Feuchtigkeit. Der von dem Aerosol befreite Gasstrom wird an der Unterseite der Trennwand 22 umgelenkt und tritt nunmehr in das Opfermetallfilter 3 ein. Es sei angenommen, daß das Gas für die Feststellung des SO₂-Gehalts aufbereitet werden soll und daß es vermutlich einen nicht unbeträchtlichen Anteil an SO₃ enthält, der entfernt werden muß. Der SO₃-Gehalt des Gases setzt sich unter Verbrauch des Metalls der Stahlwollepackung im Opfermetallfilter 3 um und wird auf diese Weise abgeschieden. In dem Membran-Feinfilter 4 werden schließlich Fein- und Feinstpartikel, z.B. Ruß, aus dem nunmehr weitgehend gereinigten Gasstrom abgeschieden, die nicht bereits vorher in den vorangehenden Ausscheidungsvorgängen entfernt worden sind.

Die Durchsatzmenge an Gas durch die gesamte Einrichtung ist von vornherein mittels des Mengenregelventils 5 und des Meßgerätes 6 so eingestellt worden, daß der anschließende Permeationstrockner 8 nicht überlastet ist. Das Gas enthält nach Verlassen des Meßgerätes 6 und der Saugpumpe 7 immer noch eine Wasserdampf-Restmenge von etwa 5-8 g/m³. Diese Restmenge diffundiert durch den inneren Schlauch 30 des Permeationstrockners 8 als Wasserdampf in die im Gegenstrom dazu strömende Luft, die von der Saugpumpe 10 durch den Einlaß 32 angesaugt und durch den äußeren Schlauch 31 gefördert wird. Am Auslaß 9 des Permeationstrockners 8 tritt nunmehr völlig von Wasserdampf und - im vorliegenden Ausführungsbeispiel - von SO₃ befreites Gas aus.

Die erfindungsgemässe Einrichtung kann auf sehr kleinem Raum untergebracht werden, z.B. in einem tragbaren Koffer, da die einzelnen Komponenten davon nur wenig Platz beanspruchen. Dies gilt insbesondere für den Gaskühler 1 in der in Fig. 2 dargestellten Ausführungsform, der besonders klein baut. Das Gesamtvolumen der Einzelgeräte und Leitungen in der erfindungsgemässen Einrichtung, das durch das zu analysierende Gas ausgefüllt werden muß, kann hierdurch bis auf 0,2 l gesenkt werden.

Wie bereits vorstehend erwähnt, enthält die erfindungsgemässe Einrichtung noch weitere Filter, ggf. Opfermetallfilter, wenn noch andere chemische Bestandteile aus dem zu analysierenden Gas entfernt werden müssen. Solche Filter können, ohne daß dies in dem vorstehend beschriebenen Ausführungsbeispiel gezeigt ist, von vornherein und ständig vorgesehen sein, auch wenn nicht unbedingt damit gerechnet werden muß, daß in dem zu untersuchenden Gas ein entsprechender chemischer Bestandteil vorliegt. In diesem Fall bedarf es nicht erst einer individuellen Anpassung an mögliche oder vermutete Gasbestandteile.

Es versteht sich, daß aufgrund der chemischen Aggressivität der in Abgasen enthaltenen Bestandteile sämtliche vorstehend beschriebenen Komponenten und Leitungen der erfindungsgemässen Einrichtung aus säurebeständigem Werkstoff, z.B. Glas, bestehen oder zumindest entsprechend beschichtete Flächen aufweisen.

So besteht beispielsweise der Gaskühler 1 - selbstverständlich mit Ausnahme des Peltier-Kühlelements - vollständig aus Glas, wobei der Durchmesser des Mantels 15 etwa bei 25 mm liegt.

## Patentansprüche

1. Kontinuierliches Verfahren zur Aufbereitung eines zu analysierenden Gases, bei dem aus einem Gasstrom in dem Gas enthaltener Wasserdampf und sonstige dampfförmige Stoffe durch Abkühlung unter den entsprechenden Taupunkt abgeschieden, das entstehende Kondensat kontinuierlich entfernt und weitere für die Analyse unerwünschte Bestandteile ausgefiltert werden, wobei anschließend an die Abscheidung von Wasserdampf durch Abkühlung und dessen Entfernung als Kondensat von vorneherein enthaltenes und während der Abkühlung als Aerosol ausfallendes Wasser ausfiltriert und schließlich das Gas durch Permeationsdestillation getrocknet wird.

2. Verfahren nach Anspruch 1,
**dadurch gekennzeichnet,**
daß anschließend an die Entfernung des Aerosols SO₃ und Halogenide durch eine chemische Reaktion abgetrennt werden.

3. Verfahren nach Anspruch 1 oder 2,
**dadurch gekennzeichnet,**
daß das durch Abkühlung erhaltene Kondensat kontinuierlich abgesaugt wird.

4. Einrichtung zur Aufbereitung eines zu analysierenden Gases, mit einem Kühler (1) mit Kondensatablauf (11) zur Kondensation von in dem Gas enthaltenem Wasserdampf und mit einem Filter (3, 4) zur Abtrennung von für die Analyse unerwünschten weiteren Bestandteilen des Gases,
dadurch gekennzeichnet,
daß dem Kühler (1) ein Aerosolfilter (2) zur Ausfiltrierung des Wasser-Aerosols nachgeschaltet ist und in Gaslaufrichtung hinter dem Aerosolfilter (2) ein Permeationstrockner (8) angeordnet ist.

5. Einrichtung nach Anspruch 4,
**dadurch gekennzeichnet,**
daß das Aerosolfilter (2) ein Feuchtigkeit aufnehmendes Fasermaterial, z.B. Baumwollfasern, enthält.

6. Einrichtung nach Anspruch 4 oder 5,
**dadurch gekennzeichnet,**
daß dem Aerosolfilter (2) mindestens ein Opfermetallfilter (3) nachgeschaltet ist.

7. Einrichtung nach Anspruch 6,
**dadurch gekennzeichnet,**
daß das Opfermetallfilter (3) Stahlwolle zur Beseitigung von SO₃ enthält.

8. Einrichtung nach Anspruch 6 oder 7,
**dadurch gekennzeichnet,**
daß das Opfermetallfilter (3) zur Beseitigung von Halogeniden Bronze oder Zink enthält.

9. Einrichtung nach einem der Ansprüche 4 bis 8,
**dadurch gekennzeichnet,**
daß an den Kondensatablauf (18), des Kühlers (1) eine Saugpumpe (13) angeschlossen ist, die in einen Kondensatbehälter (14) fördert.

10. Einrichtung nach Anspruch 9,
**dadurch gekennzeichnet,**
daß zwischen dem Kühler (1) und der Vakuum-Saugpumpe (13) eine Kondensatfalle (12) mit einem für das Kondensat durchlässigen eingangsseitigen Abschirmelement (19) angeordnet ist.

11. Einrichtung nach einem der Ansprüche 4 bis 9,
**dadurch gekennzeichnet,**
daß der Kühler (1) zumindest ein konzentrisch zu einem gekühlten Mantel (15) angeordnetes Innenrohr (16) aufweist, das mit dem Kühlereinlaß (17) verbunden ist und vor dem Kühlerauslaß im Inneren des Mantels (15) kurz vor dem Kondensatablauf (18) des Kühlers (1) mündet.

## Claims

1. A continuous process for the preparation of a gas to be analysed, wherein water vapour contained in the gas of a gas flow and other substances in vapour form are separated out of the gas flow by cooling to below the corresponding dew point, the condensate formed is continuously removed and further constituents which are undesirable for the analysis operation are filtered out, wherein following the separation of water vapour by cooling and the removal thereof as condensate water which was contained in the gas from the outset and which precipitates as an aerosol during the cooling operation is filtered out and finally the gas is dried by permeation distillation.

2. A process according to claim 1 characterised in that following the removal of the aerosol SO₃ and halides are separated off by a chemical reaction.

3. A process according to claim 1 or claim 2 characterised in that the condensate obtained by cooling is continuously sucked away.

4. Apparatus for preparing a gas to be analysed, comprising a cooler (1) with condensate discharge (11) for condensation of water vapour contained in the gas and a filter (3, 4) for separating off further constituents of the gas, which are undesirable for the analysis operation, characterised in that disposed downstream of the cooler (1) is an aerosol filter (2) for filtering out the water aerosol and arranged downstream of the aerosol filter (2) in the gas flow direction is a permeation drier (8).

5. Apparatus according to claim 4 characterised in that the aerosol filter (2) contains a moisture-absorbing fibre material, for example cotton fibres.

6. Apparatus according to claim 4 or claim 5 characterised in that at least one sacrificial metal filter (3) is disposed downstream of the aerosol filter (2).

7. Apparatus according to claim 6 characterised in that the sacrificial metal filter (3) contains steel wool for the elimination of SO₃.

8. Apparatus according to claim 6 or claim 7 characterised in that the sacrificial metal filter (3) contains bronze or zinc for the elimination of halides.

9. Apparatus according to one of claims 4 to 8 characterised in that connected to the condensate discharge (18) of the cooler (1) is a suction pump (13) which delivers into a condensate container (14).

10. Apparatus according to claim 9 characterised in that disposed between the cooler (1) and the vacuum suction pump (13) is a condensate trap (12) with a screening element (19) on its intake side, which is permeable for the condensate.

11. Apparatus according to one of claims 4 to 9 characterised in that the cooler (1) has at least one inner pipe (16) which is arranged concentrically with respect to a cooled casing (15) and which is connected to the cooler inlet (17) and which opens upstream of the cooler outlet in the interior of the casing (15) shortly upstream of the condensate outlet (18) of the cooler (1).

## Revendications

1. Procédé continu de traitement d'un gaz à analyser, dans lequel, à partir d'un courant gazeux, de la vapeur d'eau contenue dans le gaz et d'autres substances en forme de vapeur sont séparées par refroidissement en dessous du point de rosée correspondant, le produit de condensation formé étant éliminé de manière continue et d'autres éléments indésirables pour l'analyse étant séparés par filtration, l'eau contenue dès le départ et précipitant pendant le refroidissement sous la forme d'un aérosol étant, à la suite de la séparation de la vapeur d'eau par refroidissement et de son élimination sous la forme d'un produit de condensation, séparée par filtration et le gaz étant enfin séché par distillation par perméation.

2. Procédé suivant la revendication 1, caractérisé en ce que, à la suite de l'élimination de l'aérosol, du SO₃ et des halogénures sont isolés par une réaction chimique.

3. Procédé suivant l'une des revendications 1 et 2, caractérisé en ce que le produit de condensation obtenu par refroidissement est aspiré de manière continue.

4. Dispositif de traitement d'un gaz à analyser, comprenant un dispositif de refroidissement (1) pourvu d'une sortie de produit de condensation (11) pour la condensation de la vapeur d'eau contenue dans le gaz et un filtre (3, 4) pour la séparation des autres éléments du gaz qui sont indésirables pour l'analyse, caractérisé en ce qu'en aval du dispositif de refroidissement (1) est monté un filtre à aérosol (2) pour filtrer l'aérosol d'eau et en ce qu'un dispositif de séchage par perméation (8) est agencé en aval du filtre à aérosol (2), dans le sens d'écoulement du gaz.

5. Dispositif suivant la revendication 4, caractérisé en ce que le filtre à aérosol (2) contient une matière fibreuse absorbant l'humidité, par exemple des fibres de coton.

6. Dispositif suivant l'une des revendications 4 et 5, caractérisé en ce qu'au moins un filtre métallique sacrificiel (3) est monté en aval du filtre à aérosol (2).

7. Dispositif suivant la revendication 6, caractérisé en ce que le filtre métallique sacrificiel (3) contient de la laine d'acier pour l'élimination de SO₃.

8. Dispositif suivant l'une des revendications 6 et 7, caractérisé en ce que le filtre métallique sacrificiel (3) contient du bronze ou du zinc pour l'élimination d'halogénures.

9. Dispositif suivant l'une des revendications 4 à 8, caractérisé en ce qu'une pompe aspirante (13), qui refoule dans un réservoir à produit de condensation (14), est raccordée à la sortie de produit de condensation (18) du dispositif de refroidissement (1).

10. Dispositif suivant la revendication 9, caractérisé en ce qu'entre le dispositif de refroidissement (1) et la pompe aspirante sous vide (13) est agencé un piège à produit de condensation (12) présentant un élément d'écran (19) du côté entrée qui est perméable pour le produit de condensation.

11. Dispositif suivant l'une des revendications 4 à 9, caractérisé en ce que le dispositif de refroidissement (1) présente au moins un tube interne (16), qui est agencé de manière concentrique à une enveloppe (15) refroidie et est relié à l'entrée (17) du dispositif de refroidissement et qui, avant la sortie du dispositif de refroidissement, débouche à l'intérieur de l'enveloppe (15) peu en amont de la sortie de produit de condensation (18) du dispositif de refroidissement (1).
